# FASCICULE DE BREVET EUROPEEN

(11) **EP 0 804 450 B1**
(45) Date de publication et mention de la délivrance du brevet: **30.05.2001**
(21) Numéro de dépôt: 96939119.2
(22) Date de dépôt: 14.11.1996
(51) Int. Cl.: C07H 15/04

(54) **NOUVEAU PROCEDE DE FABRICATION DU PALATINITOL**
VERFAHREN ZUR HERSTELLUNG VON PALATINITOL
NOVEL METHOD FOR MAKING PALATINITOL

(30) Priorité: 17.11.1995 FR 9513647
(43) Date de publication de la demande: 05.11.1997
(73) Titulaire: Roquette Frères, 62136 Lestrem (FR)
(72) Inventeur: DUFLOT, Pierrick, F-62136 Richebourg (FR); FOUACHE, Catherine, F-62113 Sailly-Labourse (FR)
(74) Mandataire: Boulinguiez, Didier
(86) Numéro de dépôt international: FR9601798
(87) Numéro de publication internationale: WO9719094

(56) Documents cités:
- DE-A- 3 403 973
- DATABASE WPI Section Ch, Week 9225 Derwent Publications Ltd., London, GB; Class B03, AN 92-203078 XP002010687 & JP 04 121 198 A (SHOWA DENKO KK) , 22 Avril 1992
- JOURNAL OF THE AMERICAN CHEMICAL SOCIETY, vol. 74, 20 Février 1952, DC US, pages 1062-1064, XP002030029 WOLFROM M.L. ET AL: "Isomaltitol"

## Description

La présente invention concerne un nouveau procédé de fabrication du palatinitol.

Elle concerne plus particulièrement un procédé de fabrication du palatinitol à partir de l'isomaltose ou α-D-glucopyranosyl-(1→6)-D-glucose.

Le palatinitol est un édulcorant de masse peu calorique et peu cariogène que l'on obtient jusqu'à présent, par hydrogénation catalytique à pH neutre de l'isomaltulose ou α-D-glucopyranosyl-(1→6)-D-fructose, comme décrit dans la demande de brevet DE-A-34 030973.

L'isomaltulose est lui même obtenu par isomérisation enzymatique, à l'aide d'une saccharose glysosyl transférase, du saccharose ou α-D-glucopyranosyl-(1→2)-β-D-fructofuranoside.

C'est donc le saccharose qui constitue la matière première d'obtention du palatinitol, mélange dans des proportions grossièrement équimoléculaires, de α-D-glucopyranosyl (1→6)-D-sorbitol (GPS ou isomaltitol) et de α-D-glucopyranosyl-(1-6)-D-mannitol (GPM).

Le palatinitol, également dénommé isomalt, est notamment commercialisé par la Société Süddeutsche Zucker AG sous le nom Palatinit®.

On pourra se référer, entre autres documents qui concernent l'obtention et les propriétés du palatinitol, à l'ouvrage "Alternative Sweeteners" édité en 1986 par LYN O'BRIEN NABORS, chapitre 11, pages 217 à 244.

Soucieuse de développer un procédé qui permette d'obtenir le palatinitol à partir d'une autre matière première que le saccharose, la Société Demanderesse a constaté que ce but pouvait être atteint par un procédé mettant en oeuvre l'isomaltose ou α-D-glucopyranosyl-(1→6)-D-glucose.

Conformément à la présente invention, on obtient le palatinitol grâce à un procédé caractérisé par le fait que
- dans une première étape on procède à l'épimérisation de l'isomaltose dans des conditions permettant d'obtenir un mélange de α-D-glucopyranosyl-(1→6)-D-mannose et d'isomaltose,
- dans une deuxième étape on procède à l'appauvrissement en isomaltose de ce mélange épimérisé de manière à obtenir un mélange contenant une proportion grossièrement équimoléculaire de α-D-glucopyranosyl-(1→6)-D-mannose et d'isomaltose,
- dans une troisième étape, on procède à l'hydrogénation catalytique de ce mélange.

S'il est raisonnable d'imaginer que le palatinitol puisse être obtenu à partir de saccharose, l'homme du métier ne pouvait pas du tout s'attendre à ce que ce même palatinitol puisse être obtenu à partir d'isomaltose, lequel est obtenu à partir de glucose et donc d'amidons divers et variés.

En effet dans le premier cas, le saccharose dont la formule développée comprend un motif fructose donnera, de façon connue par isomérisation enzymatique, le cétose correspondant, soit l'isomaltulose.

Et il est connu de l'homme du métier que l'hydrogénation d'un tel cétose conduit à la formation des deux itols correspondants dans des proportions sensiblement équimoléculaires. Ainsi, le fait que la formule du saccharose s'apparente à celle du palatinitol laissait présumer du résultat.

Par contre, le procédé de l'invention ne met pas du tout en oeuvre un produit de départ dont la formule s'apparente à celle du palatinitol recherché. En effet, tant l'isomaltose que le glucose ou l'amidon ont une structure ne comportant pas de motif fructose et donc très éloignée de celle du palatinitol.

Le procédé de l'invention permet donc de s'affranchir de l'obligation d'utiliser le saccharose comme matière première de la fabrication du palatinitol puisque l'isomaltose peut être facilement obtenu à partir de glucose et donc d'amidons divers et variés, qu'ils soient issus de céréales ou de tubercules.

Un procédé d'obtention de l'isomaltose à partir du glucose ou d'un sirop de maïs est par exemple décrit dans la demande de brevet français FR-A-2 515 186.

Dans le procédé de l'invention, on préfère mettre en oeuvre de l'isomaltose cristallisé, quoique des sirops très riches en isomaltose conviennent également si l'on admet que du maltitol ou de l'isomaltotriitol peuvent être présents dans le palatinitol. Ces deux derniers composés proviennent de l'hydrogénation du maltose ou de l'isomaltotriose qui représentent les impuretés dominantes des sirops très riches en isomaltose.

Dans le procédé de l'invention, l'épimérisation de l'isomaltose peut être réalisée comme décrit dans la demande de brevet japonais: JP-A-63 162 698 à l'aide d'un sel métallique et d'une amine mais elle est de préférence conduite de la manière qui a été décrite dans la demande de brevet japonais 63-96195 et qui consiste à faire réagir à un pH compris entre 2,5 et 4, en présence d'anhydride molybdique ou de sels de molybdène hexavalent, à une température comprise entre 90°C et 140°C, une solution aqueuse d'isomaltose.

De préférence, on emploie le molybdate d'ammonium en une proportion d'environ 0,1 à 1,5 % en poids par rapport à l'isomaltose.

De préférence encore, on procède à l'épimérisation de l'isomaltose sous forme d'une solution sucrée aqueuse contenant de 10 à 70 % d'isomaltose.

On ajuste les conditions d'épimérisation (essentiellement taux de catalyseur, durée de l'épimérisation et température de réaction), de façon à obtenir un mélange d'isomaltose et de α-D-glucopyranosyl- (1→6)-D-mannose, contenant de 10 à 40 % de ce dernier composé. Des mélanges en contenant moins de 10 % ne sont pas économiques à traiter et des mélanges en contenant plus de 40 % contiennent trop d'impuretés qui se forment dans les conditions extrêmes d'épimérisation.

On préfère travailler dans des conditions qui permettent d'obtenir 20 à 35 % de α-D-glucopyranosyl-(1→6)-D-mannose et encore plus préférentiellement, de 25 à 35 % de ce composé.

Le mélange ainsi obtenu est alors déminéralisé sur des résines échangeuses d'ions pour en ôter les sels ayant servi de catalyseur.

Dans le procédé de l'invention, l'appauvrissement en isomaltose du mélange épimérisé peut être obtenu de différentes manières.

On peut, par exemple soumettre le mélange épimérisé à l'action d'une amyloglucosidase ce qui a pour effet d'hydrolyser l'isomaltose en deux molécules de glucose. On peut ensuite éliminer ce glucose sous forme d'acide gluconique par l'action d'une glucose oxydase qui transforme ce glucose en acide gluconique que l'on élimine par échange d'ions sur des résines anioniques. On peut aussi consommer ce glucose à l'aide de levures ou de bactéries.

On préfère toutefois procéder à l'appauvrissement en isomaltose du mélange épimérisé par voie chromatographique.

D'ordinaire, lorsque l'on fait appel à une étape chromatographique devant mener à la séparation des deux composants d'un mélange binaire, on conduit la chromatographie de manière à ce que les deux composants soient séparés de la façon la plus complète possible, c'est à dire de manière à obtenir une fraction A ne contenant que très peu de composant B et une fraction B ne contenant que très peu de composant A.

Dans le procédé de l'invention, l'appauvrissement du mélange épimérisé en isomaltose est au contraire conduit de manière à obtenir une fraction contenant une proportion grossièrement équimoléculaire d'isomaltose et de α-D-glucopyranosyl-(1-6)-D-mannose, l'autre fraction étant elle constituée d'isomaltose très pur. Par grossièrement équimoléculaire, on entend de 40 à 60 % et plus préférentiellement, de 45 à 55 % d'un des deux composés par rapport à la masse totale des deux composés. Cette manière de faire présente l'avantage d'obtenir directement un mélange qui après hydrogénation fournira les deux composants du palatinitol, à savoir l'isomaltitol et le GPM dans des proportions grossièrement équimoléculaires et ce, sans devoir avoir recours à des remélanges de fractions pures.

Avantageusement, on procède au recyclage de la fraction d'isomaltose dont la richesse est couramment comprise entre 85 % et 95 %, le reste étant surtout constitué par le α-D-glucopyranosyl-(1→6)-D-mannose, au niveau de l'étape d'épimérisation.

Cette manière de faire permet d'obtenir des rendements proches de 100 % en palatinitol obtenu par rapport à l'isomaltose mis en oeuvre et constitue donc le mode préféré de réalisation du procédé de la présente invention.

Cette étape chromatographique est menée très facilement à l'échelle industrielle par application du mélange épimérisé sur une colonne chargée avec des résines échangeuses de cations du type polystyrène sulfoné réticulé au divinylbenzène. Ces résines, pour être adaptées à la chromatographie doivent posséder une granulométrie très fine et très homogène, avantageusement comprise entre 150 et 400 microns, et pour leur utilisation, sont permutées sous forme alcaline ou alcalino-terreuse. Le mélange appliqué sur la colonne est ensuite fractionné par élution à l'eau de la résine.

On constate alors de façon surprenante, que bien que l'isomaltose et le α-D-glucopyranosyl-(1→6)-D-mannose aient des structures analogues et des poids moléculaires strictement identiques, une migration beaucoup plus rapide de l'isomaltose au sein de la résine.

Il suffit dès lors de soutirer de la résine au début du cycle d'élution, la quantité d'isomaltose strictement nécessaire pour amener dans une proportion grossièrement stoechiométrique les composants du mélange soumis à la chromatographie.

Cette étape de chromatographie peut être menée de façon discontinue sur une seule colonne de résine ou sur plusieurs colonnes fonctionnant de manière parallèle, mais elle est plus avantageusement conduite sur des systèmes multicolonnes branchées en boucle, fonctionnant selon le principe du lit mobile simulé. Ces systèmes ont l'avantage de tirer de meilleures performances de la résine et de fonctionner de manière continue.

D'une manière générale, pour obtenir les meilleures performances des résines de chromatographie, on préfère réaliser cette chromatographie à une température comprise entre 60 et 90°C. On préfère chromatographier des mélanges épimérisés ayant une matière sèche comprise entre 7 et 70 %, de préférence comprise entre 10 et 50 %. Comme il a déjà été dit plus haut, la fraction exclue en début du cycle d'élution, riche en isomaltose, est avantageusement recyclée à l'étape d'épimérisation. La fraction adsorbée, représentant la fin du cycle d'élution et contenant dans des proportions grossièrement stoechiométriques le reste de l'isomaltose et le α-D-glucopyranosyl-(1→6)-D-mannose, est alors concentrée à une matière sèche d'environ 30 % à 60 % en vue de son hydrogénation catalytique dans des conditions économiques.

Une telle hydrogénation est effectuée de manière connue en soi, continue ou discontinue, sous une pression d'hydrogène de 30 à 200 bars, à une température de 80 à 150 °C en présence de catalyseur à base de nickel ou de ruthénium et à un pH proche de la neutralité. Une hydrogénation conduite à un pH inférieur à 4,0 aurait pour résultat d'hydrolyser partiellement l'isomaltose en glucose et le α-D-glucopyranosyl-(1-6)-D-mannose en glucose et mannose avec apparition de sorbitol et de mannitol dans le palatinitol. Une hydrogénation à un pH supérieur à 9 aurait pour résultat de changer la stoechiométrie entre les deux composants du palatinitol.

De manière générale, l'hydrogénation est poursuivie jusqu'à ce que la teneur en sucres réducteurs, mesurée par la méthode de Bertrand, devienne inférieure à 1 % et de préférence, inférieure à 0,5 %.

Après l'étape d'hydrogénation, on purifie les sirops obtenus pour en ôter le catalyseur, par filtration puis déminéralisation sur résines échangeuses d'ions.

Les sirops hydrogénés et purifiés sont ensuite concentrés, cristallisés et séchés pour fournir une poudre commerciale de palatinitol qui est en fait un mélange en proportions grossièrement équimoléculaires de cristaux d'isomaltitol anhydre et de GPM dihydrate.

La présente invention est illustrée par l'exemple qui suit et qui n'est pas limitatif, la Demanderesse n'ayant pour but que d'exposer ce qui lui paraît être l'un des meilleurs moyens de mettre en oeuvre le procédé de son invention.

### EXEMPLE

### Première étape :

On met en solution dans 36 grammes d'eau, 4 grammes d'isomaltose cristallisé ainsi que 16mg de molybdate d'ammonium (NH₄)₆ Mo₇ O₂₄, soit 0,4 % en poids par rapport à l'isomaltose puis on ajuste le pH de cette solution à 3,5 à l'aide d'acide chlorhydrique.

On porte ensuite cette solution à 130°C durant 15 minutes.

Après refroidissement, on déminéralise cette solution sur un lit mixte de résines cationique et anionique fortes ce qui fournit un mélange épimérisé dont la résistivité est supérieure à 2.10⁶ ohms.cm.

Une chromatographie par HPLC de ce mélange épimérisé révèle la présence de 35 % de α-D-glucopyranosyl-(1→6)-D-mannose et de 65 % d'isomaltose. On observe aussi, quoiqu'à l'état de traces, la présence de glucose et de mannose.

### Deuxième étape :

Dans une colonne de verre à double enveloppe thermostatée à 85°C, d'une hauteur de 2 mètres et d'un diamètre intérieur de 15 mm, on introduit 340 cm³ de la résine commercialisée sous le nom de marque PCR 532 par la Société PUROLITE. Cette résine présente les caractéristiques suivantes :
- squelette : polystyrène sulfoné réticulé au divinylbenzène
- taux de réticulation : 5 %
- granulométrie : 180 à 280 microns
- forme ionique pour l'utilisation : Ca ++

On introduit au sommet de la résine, 2,5 cm³ du mélange épimérisé à 10 % de matières sèches que l'on percole au travers de cette résine en les poussant avec de l'eau, à un débit de 210 cm³/heure.

Après avoir élué 140 cm³ d'eau, on commence à recueillir une fraction d'isomaltose représentant 27 cm³.

Cette fraction d'isomaltose, montre à l'analyse chromatographique en phase gazeuse, une richesse de 89 % d'isomaltose et de 11 % de α-D-glucopyranosyl-(1→6)-D-mannose.

Immédiatement à la suite de cette fraction d'isomaltose, on récupère une fraction de 64 cm³ constituée par un mélange appauvri en isomaltose dont l'analyse chromatographique en phase gazeuse révèlera une richesse de 47 % d'isomaltose et de 52 % de α-D-glucopyranosyl-(1→6)-D-mannose. Cette analyse révèle également des traces de glucose et de mannose.

On effectue 15 fois cette étape pour obtenir une fraction d'isomaltose de richesse moyenne de 90 % et une fraction d'un mélange à parts à peu près égales d'isomaltose et de α-D-glucopyranosyl-(1→6)-D-mannose.

La fraction chromatographique exclue, riche en isomaltose a été concentrée sous vide jusqu'à une matière sèche de 10 %.

On y a à nouveau ajouté 0,4 % de molybdate d'ammonium par rapport à sa matière sèche estimée et on l'a à nouveau soumise à l'épimérisation dans les conditions décrites à propos de l'étape d'épimérisation n° 1. On a obtenu encore une fois, 35 % de α-D-glucopyranosyl-(1→6)-D-mannose.

La fraction chromatographique adsorbée, qui contient autant d'isomaltose que de α-D-glucopyranosyl-(1-6)-D-mannose a été concentrée à une matière sèche de 40 % en vue de subir l'hydrogénation.

Cette fraction, compte non tenu de la fraction d'isomaltose recyclée à l'étape d'épimérisation, représente 64 % de la matière sèche soumise au fractionnement chromatographique.

### Troisième Etape :

On a introduit cette fraction adsorbée dans un réacteur d'hydrogénation en présence de 5 % en poids des sucres, de nickel de Raney. Après avoir mis l'appareil sous une pression d'hydrogène de 50 bars que l'on maintiendra durant toute la durée de l'hydrogénation, on chauffe son contenu à la température de 125°C. On maintient tout au cours de cette hydrogénation le pH du milieu réactionnel à 8,0 à l'aide d'une solution de bicarbonate de soude. On arrête l'hydrogénation après 8 heures, alors que la teneur en sucres réducteurs du milieu réactionnel, mesurée par la méthode de Bertrand est devenue inférieure à 0,1 %.

Le contenu du réacteur d'hydrogénation est alors filtré pour en enlever le catalyseur puis le sirop est déminéralisé sur un lit mixte de résines, comme lors de la première étape. On obtient alors un sirop parfaitement limpide et incolore dont la composition par analyse chromatographique en phase gazeuse s'avère la suivante :
isomaltitol : 49,1 %
GPM : 49,4 %

Ce sirop est alors concentré de manière à amener ses deux composants à l'état de cristaux qui sont ensuite séchés pour obtenir une poudre blanche et non hygroscopique de palatinitol titrant 5,1 % d'humidité.

## Revendications

1. Procédé de fabrication du palatinitol, caractérisé par le fait que
- dans une première étape, on procède à l'épimérisation de l'isomaltose dans des conditions permettant d'obtenir un mélange de α-D-glucopyranosyl-(1→6)-D-mannose et d'isomaltose,
- dans une deuxième étape, on procède à l'appauvrissement en isomaltose de ce mélange épimérisé de manière à obtenir un nouveau mélange contenant une proportion grossièrement équimoléculaire de α-D-glucopyranosyl-(1→6)-D-mannose et d'isomaltose,
- dans une troisième étape, on procède à l'hydrogénation catalytique de ce mélange grossièrement équimoléculaire.

2. Procédé selon la revendication 1, caractérisé par le fait que l'on procède à l'épimérisation en présence d'un sel de molybdène hexavalent.

3. Procédé selon l'une ou l'autre des revendications 1 et 2, caractérisé par le fait que l'appauvrissement du mélange épimérisé en isomaltose est effectué par chromatographie sur des résines cationiques sous forme alcaline ou alcalino-terreuse.

4. Procédé selon la revendication 3, caractérisé par le fait que les résines cationiques sont sous forme calcium.

5. Procédé selon l'une ou l'autre des revendications 3 et 4, caractérisé par le fait que la fraction d'isomaltose exclue de l'étape de chromatographie est recyclée à l'étape d'épimérisation.

## Patentansprüche

1. Verfahren zur Herstellung von Palatinitol, dadurch gekennzeichnet, daß man
- in einer ersten Stufe die Epimerisierung von Isomaltose unter Bedingungen durchführt, die es ermöglichen, eine Mischung von α-D-Glucopyranosyl-(1→6)-D-Mannose und Isomaltose zu erhalten,
- in einer zweiten Stufe die Anreicherung von Isomaltose mit dieser epimerisierten Mischung in der Weise durchführt, daß man eine neue Mischung erhält, die ein etwa äquimolekulares Verhältnis von α-D-Glucopyranosyl-(1→6)-D-Mannose und Isomaltose enthält,
- in einer dritten Stufe die katalytische Hydrierung mit dieser etwa äquimolekularen Mischung durchführt.

2. Verfahren nach Anspruch 1, dadurch gekennzeichnet, daß man die Epimerisierung in Anwesenheit eines hexavalenten Molybdänsalzes durchführt.

3. Verfahren nach dem einen oder anderen der Ansprüche 1 und 2, dadurch gekennzeichnet, daß die Anreicherung dieser epimerisierten Mischung an Isomaltose mittels Chromatographie auf kationischen Harzen in der Form Alkali oder Erdalkali durchgeführt wird.

4. Verfahren nach Anspruch 3, dadurch gekennzeichnet, daß die kationischen Harze in der Form Calcium vorliegen.

5. Verfahren nach dem einen oder anderen der Ansprüche 3 und 4, dadurch gekennzeichnet, daß die Fraktion von Isomaltose von der Stufe der Chromatographie ausgeschlossen und zur Stufe der Epimerisierung zurückgeführt wird.

## Claims

1. Process for the preparation of palatinitol, characterized in that:
- in a first stage, the epimerization of isomaltose is carried out under conditions which allow a mixture of α-D-glucopyranosyl-(1→6)-D-mannose and isomaltose to be obtained,
- in a second stage, the epimerized mixture is depleted of isomaltose so as to obtain a new mixture containing a roughly equimolecular proportion of α-D-glucopyranosyl-(1→6)-D-mannose and isomaltose,
- in a third stage, catalytic hydrogenation is carried out on this roughly equimolecular mixture.

2. Process according to claim 1, characterized in that the epimerization is carried out in the presence of a hexavalent molybdenum salt.

3. Process according to one or other of claims 1 and 2, characterized in that depleting the epimerized mixture of isomaltose is carried out by chromatography on cationic resins in alkaline or alkaline-earth form.

4. Process according to claim 3, characterized in that the cationic resins are in calcium form.

5. Process according, to one or other of claims 3 and 4, characterized in that the fraction of isomaltose excluded from the chromatographic stage is recycled at the epimerization stage.
